# EUROPEAN PATENT SPECIFICATION

(11) **EP 1 309 315 B1**
(45) Date of publication and mention of the grant of the patent: **14.06.2006**
(21) Application number: 01964122.4
(22) Date of filing: 17.08.2001
(51) Int. Cl.: A61K 9/20, A61K 31/635, A61K 31/415

(54) **RAPIDLY DISINTEGRATING ORAL FORMULATION OF VALDECOXIB**
SCHNELL ZERFALLENDE ORALE ARZNEIZUBEREITUNG ENTHALTEND VALDECOXIB
PREPARATION ORALE A DESINTEGRATION RAPIDE DE VALDECOXIB

(30) Priority: 18.08.2000 US 226487 P
(43) Date of publication of application: 14.05.2003
(73) Proprietor: Pharmacia Corporation, St. Louis, MO 63167 (US)
(72) Inventor: KARARLI, Tugrul T., Glenview, IL 60025-7787 (US); KONTNY, Mark J., Libertyville, IL 60048 (US); LE, Trang T., Mundelein, IL 60060 (US)
(74) Representative: Albrecht, Thomas
(86) International application number: PCT/US2001/025762
(87) International publication number: WO 2002/015884

(56) References cited:
- EP-A- 0 396 335
- EP-A- 0 748 628
- WO-A-00/27382
- WO-A-00/32189
- WO-A-01/39749
- WO-A-97/44028
- WO-A-99/04765
- WO-A-99/47172
- US-A- 5 466 464
- US-A- 5 501 861
- US-A- 5 733 577

## Description

### FIELD OF THE INVENTION

The present invention relates to orally deliverable pharmaceutical compositions containing a selective cyclooxygenase-2 inhibitory drug, to processes for preparing such compositions, and to the use of such compositions in the manufacture of medicaments.

### BACKGROUND OF THE INVENTION

Numerous compounds have been reported having therapeutically and/or prophylactically useful selective cyclooxygenase-2 inhibitory effect, and have been disclosed as having utility in treatment or prevention of specific cyclooxygenase-2 mediated disorders or of such disorders in general. Among such compounds are a large number of substituted pyrazolyl benzenesulfonamides as reported in U.S. Patent No. 5,760,068 to Talley *et al.,* including for example the compound 4-[5-(4-methylphenyl)-3-(trifluoromethyl)-1H-pyrazol-1-yl]benzenesulfonamide, also referred to herein as celecoxib (I), and the compound 4-[5-(3-fluoro-4-methoxyphenyl)-3-difluoromethyl)-1H-pyrazol-1-yl]benzenesulfonamide, also referred to herein as deracoxib (II).

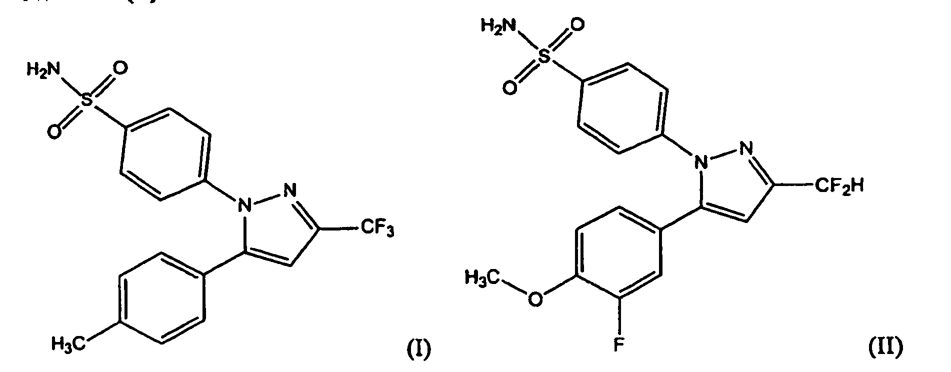

Other compounds reported to have therapeutically and/or prophylactically useful selective cyclooxygenase-2 inhibitory effect are substituted isoxazolyl benzenesulfonamides as reported in U.S. Patent No. 5,633,272 to Talley *et al*., including for example the compound 4-[5-methyl-3-phenylisoxazol-4-yl]benzenesulfonamide, also referred to herein as valdecoxib (III).

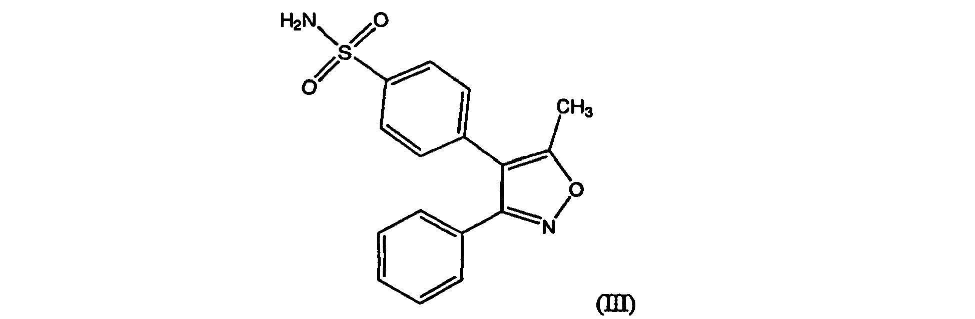

Still other compounds reported to have therapeutically and/or prophylactically useful selective cyclooxygenase-2 inhibitory effect are substituted (methylsulfonyl)phenyl furanones as reported in U.S. Patent No. 5,474,995 to Ducharme *et al*., including for example the compound 3-phenyl-4-[4-(methylsulfonyl)phenyll-5H-furan-2-one, also referred to herein as rofecoxib (IV).

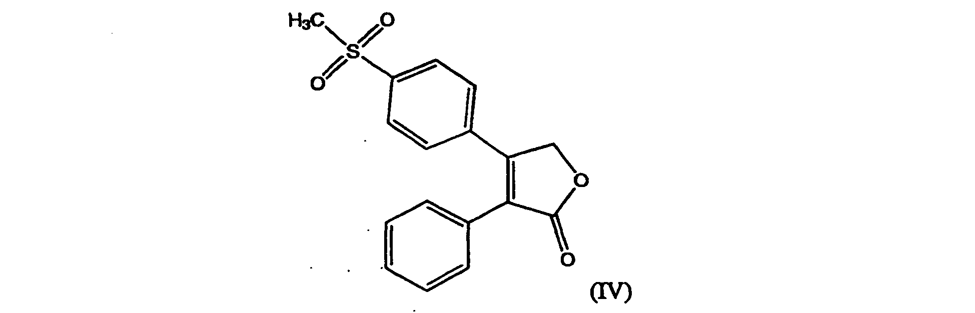

U.S. Patent No. 5,981,576 to Belley *et al*. discloses a further series of (methylsulfonyl)phenyl furanones said to be useful as selective cyclooxygenase-2 inhibitory drugs, including 3-(1-cyclopropylmethoxy)-5,5-dimethyl-4-[4-(methylsulfonyl)phenyl]-5H-furan-2-one and 3-(1-cyclopropylethoxy)-5,5-dimethyl-4-[4-(methylsulfonyl)phenyl]-5H-furan-2-one.

U.S. Patent No. 5,861,419 to Dube *et al*. discloses substituted pyridines said to be useful as selective cyclooxygenase-2 inhibitory drugs, including for example the compound 5-chloro-3-(4-methylsulfonyl)phenyl-2-(2-methyl-5-pyridinyl)pyridine, also referred to herein as etoricoxib (V).

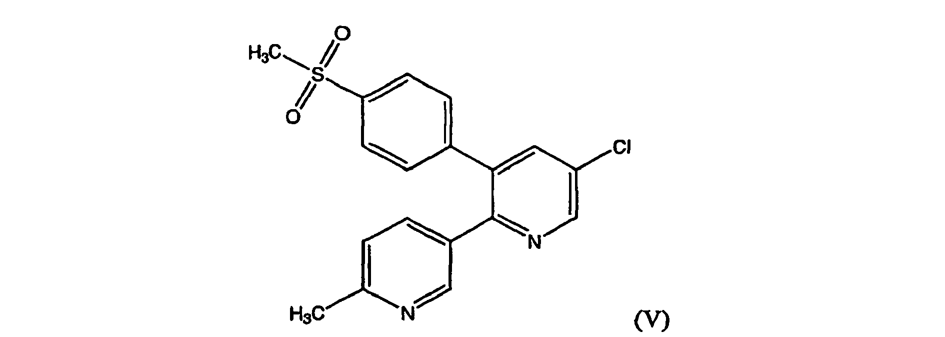

European Patent Application No. 0 863 134 discloses the compound 2-(3,5-difluorophenyl)-3-[4-(methylsulfonyl)phenyl]-2-cyclopenten-1-one said to be useful as a selective cyclooxygenase-2 inhibitory drug.

U.S. Patent No. 6,034,256 to Carter *et al*. discloses a series of benzopyrans said to be useful as selective cyclooxygenase-2 inhibitory drugs, including the compound (S)-6,8-dichloro-2-(trifluoromethyl)-2H-1-benzopyran-3-carboxylic acid (VI).

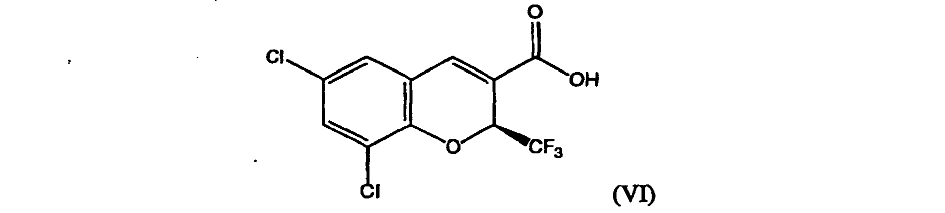

International Patent Publication No. WO 00/24.719 discloses substituted pyridazinones said to be useful as selective cyclooxygenase-2 inhibitory drugs, including the compound 2-(3,4-difluorophenyl)-4-(3-hydroxy-3-methyl-1-butoxy)-5-[4-(methylsulfonyl)phenyl]-3-(2H)-pyridazinone.

A need for formulated compositions of selective cyclooxygenase-2 inhibitory drugs, in particular, easy-to-swallow compositions, exists. Easy-to-swallow drug delivery systems can provide many benefits over conventional dosage forms, particularly to populations such as the elderly, young children and other groups of patients that have difficulty swallowing conventional oral preparations.

Common oral dosage forms such as tablets, pills or capsules generally must be swallowed with water. Many pediatric and elderly patients with weak swallowing ability are unwilling or unable to swallow such dosage forms.

Powders and granules are additional commonly used oral dosage forms. However, these formulations can be difficult to swallow completely due to their tendency to remain in the oral cavity. In some instances, patients taking powdered dosage forms will feel choked with powder or feel pain or unpleasantness due to granules being lodged under dentures. Additionally, powders and granules typically can only be used after the tearing or breaking of a package, tasks that elderly patients often find difficult to perform.

Further, powder and granule dosage forms are inconvenient to take as they typically must be diluted with a suitable amount of water or other liquid carrier prior to ingestion. This is particularly problematic when the medication is needed to provide fast relief of pain, since water is not always readily obtainable throughout the day. Moreover, powders or granules taken after dissolution or suspension in a liquid can also be difficult for elderly patients suffering from incontinence as such patients may experience urination problems at night when relatively large volumes of liquid-based medications are taken before bedtime.

Syrups and elixirs are additional commonly used oral dosage forms. However, elderly patients and others who have difficulty in measuring precise volumes are unlikely to be able to administer to themselves a proper dose and therefore require assistance at each administration.

International Patent Publication No. WO 00/32189 discloses various oral preparations of celecoxib. However, easy-to-swallow preparations of compositions containing selective cyclooxygenase-2 inhibitory drugs have not been disclosed.

In light of the expanding elderly population, it is becoming critically important to develop safe, effective, easy-to-swallow pharmaceutical preparations to treat age-related indications, wherein such preparations are convenient for elderly patients to self-administer and ingest.

U.S. Patent No. 5,576,014 discloses an intrabuccally dissolving compressed molding prepared by a wet granulation process wherein a low moldability saccharide is granulated with a high moldability saccharide to form a granulate, which is then compressed into a molding. The resulting molding can incorporate a drug and is said to show quick disintegration and dissolution in the buccal cavity but to maintain sufficient hardness so as not break during production and distribution. The compressed molding of U.S. Patent No. 5,576,014 is a type of dosage form known as a "fast-melt tablet", exhibiting rapid disintegration, usually associated with the carrier materials, typically sugars, and concomitant rapid dissolution or dispersion of the drug in the mouth, usually without need for water other than that contained in saliva. A drug formulated in such a tablet is readily swallowed.

International Patent Publication No. WO 97/44028 describes a pharmaceutical composition for the treatment of cyclooxygenase-2 mediated diseases suitable for once a day administration and comprising a cyclooxygenase-2 inhibiting compound characterized by high potency, a long half-life and a high degree of specificity for inhibiting cyclooxygenase-2 in preference to cyclooxygenase-1, in particular, 3-phenyl-4-(4-methylsulfonyl)phenyl)-2-(5*H*)-furanone. This document also discloses tablets with celecoxib (5-10% by weight) and lactose (42.5-45% by weight), prepared by direct compression.

International Patent Publication No. WO 00/27382 describes combinations of a GABA_{A} alpha 5 inverse agonist and a COX-2 inhibitor, NSAID, estrogen or vitamin E are disclosed for treating neurodegenerative conditions such as Alzheimer's Disease. This document also describes tablets with 5- 25% by weight of a cyclooxygenase-2 inhibitor (celecoxib, rofecoxib or valdecoxib) and 42-45% by weight of lactose, prepared by direct compression.

International Patent Publication No. WO 99/04765 describes a pharmaceutical composition in form of effervescent tablets comprising an active ingredient and an effervescent blend, wherein the effervescent blend comprises an acidic component and sodium glycine carbonate as alkaline components. Preferred acid component are fumaric acid, maleic acid and their salts. Tablets are prepared by direct compression in normal thermo-hygrometric conditions and with standard tabletting equipment. This document also describes a fast disintegrating effervescent tablet with rapid disintegration comprising 3.2% by weight of nimesulide, and 32% by weight of lactose which is prepared by direct compression.

European Patent Application No. 0 748 628 describes organoleptically acceptable formulations containing S(+) 1,8-diethyl-1,3,4,9-tetrahydropyrano[3,4-b]indole-1-acetic acid, preferably with an acidic component, also known as S(+)etodolic acid or S(+)etodolac. This document also describes chewable tablets with 21.8% by weight of etodolac and 58% mannitol and rapidly disintegrating tablets with 22% by weight of etodolac and 33% by weight of fructose, prepared by freeze-drying in a mold as well as a lozenge with etodolac (2.5% by weight) and sucrose and corn syrup (95% by weight).

International Patent Publication No. WO 99/47172 describes pharmaceutical compositions for oral administration which contain inclusion complexes and are characterized by rapid release of the active ingredient and the fact that they require no use of liquids for administration, being the saliva present in the oral cavity adequate for dissolution of the active ingredient. This document also discloses rapidly dissolving chewable tablets for insoluble active principles such as tablets comprising nimesulide (2.8% by weight), xylitol (44.5% by weight), and cyclodextrin (22.8% by weight) prepared by direct compression.

European Patent Application No. 0 396 335 discloses a chewable tablet comprising a medicament dispersed in a chewable base, such as mannitol, together with an effervescent couple, such as citric acid/sodium bicarbonate. This document also discloses an effervescent chewable tablet with 45% by weight of nabumetone, 15% by weight of sorbitol and 30-33% by weight of mannitol, prepared by direct compression.

The term "fast-melt" as used herein refers to a composition such as a tablet wherein an active agent or drug is distributed or dispersed in a matrix formed by a carrier that, upon oral administration to a subject, disintegrates in the oral cavity, thereby releasing the drug, typically in particulate form, for entry to the gastrointestinal tract by swallowing, and subsequent absorption. The term "oral cavity" includes the entire interior of the mouth, including not only the buccal cavity (that part of the oral cavity anterior to the teeth and gums) but also the sublingual and supralingual spaces.

With respect to drugs requiring a high dose for therapeutic effectiveness, the large size of a fast-melt tablet required to provide a therapeutic dose may be a limiting factor. To reduce tablet size, drug loading can be increased in a given formulation. However, typical fast-melt tablet formulations begin to lose their rapid disintegration characteristics as the relative amount of active agent in the tablet increases, at least in part because of the corresponding reduction in the amount of readily soluble and/or disintegratable carrier. Alternatively, several tablets having a low drug loading would have to be ingested, which can result in patient inconvenience and decreased compliance.

However, selective cyclooxygenase-2 inhibitory drugs present certain challenges for formulation as fast-melt tablets. For example, many selective cyclooxygenase-2 inhibitory compounds, including celecoxib, deracoxib, valdecoxib, 2-(3,5-difluorophenyl)-3-[4-(methylsulfonyl)phenyl]-2-cyclopenten-1-one, etoricoxib and rofecoxib, have very low solubility in aqueous media. In addition, some, for example celecoxib, have relatively high dose requirements. Celecoxib also presents difficulties as a result of unique physical and chemical characteristics such as electrostatic and cohesive properties, low bulk density, low compressibility and poor flow properties.

Due at least in part to these properties, celecoxib crystals tend to segregate and agglomerate together during mixing, resulting in a non-uniformly blended composition containing undesirably large, insoluble aggregates of celecoxib. Therefore, it is difficult to prepare a fast-melt composition containing celecoxib that has the desired blend uniformity for rapid and complete disintegration in the mouth.

It would be a much desired advance in the art to provide a fast-melt formulation of a selective cyclooxygenase-2 inhibitory drug of low solubility, such as celecoxib, that has the desired blend uniformity for rapid and complete disintegration in the mouth.

### SUMMARY OF THE INVENTION

According to the present invention, there is now provided a molded article, e.g., a tablet, for administration to an oral cavity of a subject to treat or prevent a cyclooxygenase-2 mediated condition, disorder or disease, the molded article comprising a moldable blend of a therapeutically effective amount of the selective cyclooxygenase-2 inhibitory drug valdecoxib with a pharmaceutically acceptable excipient carrier system comprising one or more carbohydrates, wherein said carbohydrates constitute more than 50% by weight of all excipients in the moldable blend, wherein ingredients and amounts thereof in the molded article and a process for preparing the molded article are selected such that the molded article exhibits rapid disintegration in the oral cavity, and wherein the moldable blend is prepared by a process step not requiring wet granulation, wherein said article disintegrates within 30 to 300 seconds after placement in a standard in vitro disintegration assay, conducted according to U.S. Pharmacopeia 24 (2000), Test No. 701, and wherein the selective cyclooxygenase-2 inhibitory drug is present in a total amount of 20% to 60% by weight of the molded article.

By selecting a process step not requiring wet granulation for preparing the blend of the drug with the excipient carrier system, one or more of the following advantages can be obtained:
(a) the overall process for preparing the molded article can be simplified, e.g., fewer steps can be required;
(b) problems during granulation arising from physical or chemical characteristics of the drug can be avoided;
(c) a more rapidly disintegrating molded article can be provided;
(d) the molded article can have improved organoleptic qualities, e.g., enhanced "mouth feel";
(e) the molded article can exhibit improved resistance to breakage or attrition during handling, packaging, removal from a package, etc.;
(f) greater flexibility can be obtained in the form, e.g., size or shape, of the molded article.

It will be understood that even if a non-essential wet granulation step is added to a process step for preparing a blend, that process step remains one "not requiring wet granulation" as defined herein. In other words, a molded article or a process for preparing it is not removed from the scope of the present invention by opportunistic inclusion in the process of a wet granulation step, except where such wet granulation step is necessary to provide a moldable blend or to provide a molded article exhibiting rapid disintegration in the oral cavity as required herein. Further, pre-preparation of a particular excipient by a process that includes wet granulation is not to be considered to remove a molded article containing that excipient from the scope of the present invention, if a wet granulation step is not required in the blending of that ingredient with others forming the molded article.

Processes suitable for preparing a molded article of the invention include, without limitation, processes substantially as disclosed in any of the patents listed below, with modification as required for an active agent that is valdecoxib. Such modification will readily be made by one of skill in the art of pharmaceutical formulation. Where patents listed below disclose various processes, some of which have a wet granulation step, it is understood that for the purposes of the present invention a process is to be selected having no requirement for such a wet granulation step.
U.S. Patent No. 3,885,026 to Heinemann & Rothe.
U.S. Patent No. 4,134,943 to Knitsch *et al*.
U.S. Patent No. 4,305,502 to Gregory & Ho.
U.S. Patent No. 4,371,516 to Gregory *et al*.
U.S. Patent No. 4,414,198 to Michaelson.
U.S. Patent No. 4,855,326 to Fuisz.
U.S. Patent No. 4,946,684 to Blank *et al*.
U.S. Patent No. 5,073,374 to McCarty.
U.S. Patent No. 5,178,878 to Wehling *et al*.
U.S. Patent No. 5,298,261 to Pebley *et al*.
U.S. Patent No. 5,401,514 to Juch *et al*.
U.S. Patent No. 5,464,632 to Cousin *et al*.
U.S. Patent No. 5,466,464 to Masaki & Ban.
U.S. Patent No. 5,082,667 to Van Scoik.
U.S. Patent No. 5,501,861 to Makino *et al*.
U.S. Patent No. 5,503,846 to Wehling *et al*.
U.S. Patent No. 5,587,172 to Cherukuri *et al*.
U.S. Patent No. 5,587,180 to Allen & Wang.
U.S. Patent No. 5,607,697 to Alkire *et al*.
U.S. Patent No. 5,622,719 to Myers *et al*.
U.S. Patent No. 5,653,926 to Bogue & Myers.
U.S. Patent No. 5,662,849 to Bogue & Myers.
U.S. Patent No. 5,733,577 to Myers *et al*.
U.S. Patent No. 5,762,961 to Roser & Blair.
U.S. Patent No. 5,807,576 to Allen *et al*.
U.S. Patent No. 5,837,285 to Nakamichi *et al*.
U.S. Patent No. 5,869,098 to Misra *et al*.
U.S. Patent No. 5,876,759 to Gowan.
U.S. Patent No. 5,939,091 to Eoga & Valia.
U.S. Patent No. 5,958,453 to Ohno *et al*.
U.S. Patent No. 6,010,719 to Remon & Corveleyn.
U.S. Patent No. 6,024,981 to Khankari *et al*.
International Patent Publication No. WO 00/47233.

Some of the above, and other, approaches to formulating fast-melt tablets have been summarized by Chang *et al*. in Pharmaceutical Technology, June 2000, pp. 52-58.

The statement herein that the excipient carrier system "consists predominantly of" one or more carbohydrates is to be understood to mean that carbohydrates constitute more than 50% by weight of all excipients in the moldable blend formed with the selective cyclooxygenase-2 inhibitory drug. Carbohydrates useful in the excipient carrier system include natural and modified celluloses, natural and modified starches, mono-, di- and oligosaccharide sugars and sugar alcohols. The term "saccharide" is used herein to denote a sugar or sugar alcohol having 1 to about 6 saccharide units. Preferred carbohydrates are saccharides; more preferred are mono- and disaccharides.

Also provided by the present invention is a process for preparing a molded article which is an oral fast-melt dosage form of the selective cyclooxygenase-2 inhibitory drug valdecoxib, the process comprising a step of intimately mixing valdecoxib in a therapeutically effective amount with an excipient carrier system comprising one or more carbohydrates, to form a blend, wherein formation of the blend does not require wet granulation and wherein said carbohydrates constitute more than 50% by weight of said blend; and a step of shaping a unit-dose quantity of the blend in a mold to form the molded article; wherein said article disintegrates within 30 to 300 seconds after placement in a standard in vitro disintegration assay, conducted according to U.S. Pharmacopeia 24 (2000), Test No. 701, and wherein the selective cyclooxygenase-2 inhibitory drug is present in a total amount of 20% to 60% by weight of the molded article.

The process illustratively follows a process substantially as described in any of the above-cited patents, with modification as appropriate for valdecoxib as active agent.

A "unit-dose quantity" herein is an amount of the moldable blend that contains an amount of the drug intended for a single administration to the mouth. Where the moldable blend is liquid or semi-liquid, as for example a paste, the shaping step can be accomplished by placement in a suitable mold and drying, for example by heat, by vacuum or by freeze-drying. Alternatively the shaping step can be accomplished by compression, for example in a tableting press.

Preferred molded articles of the invention are tablets. The articles of the invention disintegrate within about 30 to about 300 seconds after placement in a standard *in vitro* disintegration assay (*e*.*g*., conducted according to U.S. Pharmacopeia 24 (2000), Test No: 701) and/or disintegrate within about 5 to about 60 seconds after placement in the oral cavity of a subject. Preferably, such tablets have a hardness of about 1 kp to about 10 kp. Such oral fast-melt tablets provide a heretofore nonexistent dosage form of valdecoxib that is efficient to produce, convenient and easy to swallow.

Also provided by the present invention is a method of use of a composition of the invention for preparing a medicament. Other features of this invention will be in part apparent and in part pointed out hereinafter.

### DETAILED DESCRIPTION OF THE INVENTION

As indicated above, the present invention provides a process that comprises a step of intimately mixing the selective cyclooxygenase-2 inhibitory drug valdecoxib in a therapeutically effective amount with an excipient carrier system predominantly consisting of one or more carbohydrates, without wet granulating, to form a moldable blend; and a step of shaping a unit-dose quantity of the moldable blend in a mold to form a molded article. The molded article is useful as an oral fast-melt formulation of the drug, and is itself a further embodiment of the invention.

The invention is illustrated herein with particular reference to celecoxib (although the aforementioned article, process and use are not claimed in relation to celecoxib), and it will be understood that valdecoxib can be substituted for celecoxib in particular processes and molded articles herein described.

Celecoxib can be prepared by a process known *per se*, for example by processes set forth in U.S. Patent No. 5,466,823 to Talley *et al*. or in U.S. Patent No. 5,892,053 to Zhi & Newaz. Other selective cyclooxygenase-2 inhibitory drugs can be prepared by processes known *per se*, including processes set forth in patent publications disclosing such drugs; for example in the case of valdecoxib in above-cited U.S. Patent No. 5,633,272, and in the case of rofecoxib in above-cited U.S. Patent No. 5,474,995.

Celecoxib dosage forms preferably comprise celecoxib in a daily dosage amount of about 10 mg to about 1000 mg, more preferably about 25 mg to about 400 mg, and most preferably about 50 mg to about 200 mg.

For other selective cyclooxygenase-2 inhibitory drugs, a daily dosage amount can be in a range known to be therapeutically effective for such drugs. Preferably, the daily dosage amount is in a range providing therapeutic equivalence to celecoxib in the daily dosage ranges indicated immediately above.

Single molded articles wherein celecoxib is the selective cyclooxygenase-2 inhibitory drug typically contain about 10 mg to about 400 mg of celecoxib, for example, a 10, 20, 37.5, 50, 75, 100, 125, 150, 175, 200, 250, 300, 350 or 400 mg dose of celecoxib. Preferred such molded articles contain about 25 mg to about 400 mg of celecoxib. More preferred such molded articles contain about 50 mg to about 200 mg of celecoxib. A particular unit dose can be selected to accommodate the desired frequency of administration used to achieve a specified daily dosage. The amount of the unit dose that is administered and the dosage regimen for treating the condition or disorder will depend on a variety of factors, including the age, weight, sex and medical condition of the subject, the severity of the condition or disorder, the route and frequency of administration, and the particular selective cyclooxygenase-2 inhibitory drug selected, and thus may vary widely. It is contemplated, however, that for most purposes a once-a-day or twice-a-day administration regimen provides the desired therapeutic efficacy.

Valdecoxib is present in an amount of 20% to 60%, for example about 50%, by weight of the composition.

Molded articles, herein also referred to as compositions, of the present invention are useful in treatment and prevention of a very wide range of disorders mediated by cyclooxygenase-2 (COX-2), including but not restricted to disorders characterized by inflammation, pain and/or fever. Such compositions are especially useful as anti-inflammatory agents, such as in treatment of arthritis, with the additional benefit of having significantly less harmful side effects than compositions of conventional nonsteroidal anti-inflammatory drugs (NSAIDs) that lack selectivity for COX-2 over COX-1. In particular, such compositions have reduced potential for gastrointestinal toxicity and gastrointestinal irritation including upper gastrointestinal ulceration and bleeding, reduced potential for renal side effects such as reduction in renal function leading to fluid retention and exacerbation of hypertension, reduced effect on bleeding times including inhibition of platelet function, and possibly a lessened ability to induce asthma attacks in aspirin-sensitive asthmatic subjects, by comparison with compositions of conventional NSAIDs. Thus compositions of the invention comprising a selective COX-2 inhibitory drug are particularly useful as an alternative to conventional NSAIDs where such NSAIDs are contraindicated, for example in patients with peptic ulcers, gastritis, regional enteritis, ulcerative colitis, diverticulitis or with a recurrent history of gastrointestinal lesions; gastrointestinal bleeding, coagulation disorders including anemia such as hypoprothrombinemia, hemophilia or other bleeding problems; kidney disease; or in patients prior to surgery or patients taking anticoagulants.

Such compositions are useful to treat arthritic disorders, including but not limited to rheumatoid arthritis, spondyloarthmpathies, gouty arthritis, osteoarthritis, systemic lupus erythematosus and juvenile arthritis.

Such compositions are also useful in treatment of asthma, bronchitis, menstrual cramps, preterm labor, tendinitis, bursitis, allergic neuritis, cytomegalovirus infectivity, apoptosis including HTV-induced apoptosis, lumbago, liver disease including hepatitis, skin-related conditions such as psoriasis, eczema, acne, bums, dermatitis and ultraviolet radiation damage including sunburn, and post-operative inflammation including that following ophthalmic surgery such as cataract surgery or refractive surgery.

Such compositions are useful to treat gastrointestinal conditions such as inflammatory bowel disease, Crohn's disease, gastritis, irritable bowel syndrome and ulcerative colitis.

Such compositions are useful in treating inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of ophthalmic diseases, such as retinitis, scleritis, episcleritis, conjunctivitis, retinopathies, uveitis, ocular photophobia, and of acute injury to eye tissue.

Such compositions are useful in treatment of pulmonary inflammation, such as that associated with viral infections and cystic fibrosis, and in bone resorption such as that associated with osteoporosis.

Such compositions are useful for treatment of certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, bums, and trauma following surgical and dental procedures.

Such compositions are useful for, but not limited to, treating and preventing inflammation-related cardiovascular disorders in a subject. Such compositions are useful for treatment and prevention of vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful for, but not limited to, treatment of angiogenesis-related disorders in a subject, for example to inhibit tumor angiogenesis. Such compositions are useful for treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and glaucoma, including neovascular glaucoma; ulcerative diseases such as gastric ulcer; pathological, but non-malignant, conditions such as hemangiomas, including infantile hemangiomas, angiofibroma of the nasopharynx and avascular necrosis of bone; and disorders of the female reproductive system such as endometriosis.

Such compositions are useful for prevention or treatment of benign and malignant tumors/neoplasia including cancers, for example colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that affect epithelial cells throughout the body. Neoplasias for treatment of which compositions of the invention are contemplated to be particularly useful are gastrointestinal cancer, Barrett's esophagus, liver cancer, bladder cancer, pancreas cancer, ovary cancer, prostate cancer, cervical cancer, lung cancer, breast cancer and skin cancer, such as squamous cell and basal cell cancers. Compositions of the invention can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in patients at risk of FAP.

Such compositions inhibit prostanoid-induced smooth muscle contraction by preventing synthesis of contractile prostanoids and hence can be of use in treatment of dysmenorrhea, premature labor, asthma and eosinophil-related disorders. They also can be of use for decreasing bone loss particularly in postmenopausal women (i.e., treatment of osteoporosis), and for treatment of glaucoma.

Preferred uses for compositions of the present invention are for treatment of rheumatoid arthritis and osteoarthritis, for pain management generally (particularly post-oral surgery pain, post-general surgery pain, post-orthopedic surgery pain, and acute flares of osteoarthritis), for treatment of Alzheimer's disease, and for colon cancer chemoprevention.

As a reference, for treatment of rheumatoid arthritis or osteoarthritis, such compositions can be used to provide a daily dosage of celecoxib of about 50 mg to about 1000 mg, preferably about 100 mg to about 600 mg, more preferably about 150 mg to about 500 mg, still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in such a composition. The daily dose can be administered in one to about four doses per day, preferably one or two doses per day.

For treatment of Alzheimer's disease or cancer, such compositions can be used to provide a daily dosage of celecoxib of about 50 mg to about 1000 mg, preferably about 100 mg to about 800 mg, more preferably about 150 mg to about 600 mg, and still more preferably about 175 mg to about 400 mg, for example about 400 mg. A daily dose of about 0.7 to about 13 mg/kg body weight, preferably about 1.3 to about 10.7 mg/kg body weight, more preferably about 2 to about 8 mg/kg body weight, and still more preferably about 2.3 to about 5.3 mg/kg body weight, for example about 5.3 mg/kg body weight, is generally appropriate when administered in such a composition. The daily dose can be administered in one to about four doses per day, preferably one or two doses per day.

For pain management generally and specifically for treatment and prevention of headache and migraine, such compositions can be used to provide a daily dosage of celecoxib of about 50 mg to about 1000 mg, preferably about 100 mg to about 600 mg, more preferably about 150 mg to about 500 mg, and still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in such a composition. The daily dose can be administered in one to about four doses per day. Administration at a rate of one 50 mg dose unit four times a day, one 100 mg dose unit or two 50 mg dose units twice a day or one 200 mg dose unit, two 100 mg dose units or four 50 mg dose units once a day is preferred.

For valdecoxib appropriate doses can be selected by reference to the patent literature cited hereinabove.

Besides being useful for human treatment, compositions of the invention are also useful for veterinary treatment of companion animals, exotic animals, farm animals, and the like, particularly mammals including rodents. More particularly, compositions of the invention are useful for veterinary treatment of cyclooxygenase-2 mediated disorders in horses, dogs and cats.

The present invention also is related to a therapeutic method of treating a condition or disorder (not claimed as such) where treatment with a cyclooxygenase-2 inhibitory drug is indicated, the method comprising oral administration of one or more compositions of the present invention to a patient in need thereof. The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the patient and the nature and severity of the disorder. Thus, the dosage regimen actually employed can vary widely and can therefore deviate from the preferred dosage regimens set forth above.

Initial treatment of a patient suffering from a condition or disorder where treatment with a cyclooxygenase-2 inhibitory drug is indicated can begin with a dose regimen as indicated above. Treatment is generally continued as necessary over a period of several weeks to several months or years until the condition or disorder has been controlled or eliminated. Patients undergoing treatment with a composition of the invention can be routinely monitored by any of the methods well known in the art to determine the effectiveness of therapy. Continuous analysis of data from such monitoring permits modification of the treatment regimen during therapy so that optimally effective amounts of the drug are administered at any point in time, and so that the duration of treatment can be determined. In this way, the treatment regimen and dosing schedule can be rationally modified over the course of therapy so that the lowest amount of the drug exhibiting satisfactory effectiveness is administered, and so that administration is continued only for so long as is necessary to successfully treat the condition or disorder.

The present compositions can be used in combination therapies with opioids and other analgesics, including narcotic analgesics, Mu receptor antagonists, Kappa receptor antagonists, non-narcotic (i.e. non-addictive) analgesics, monamine uptake inhibitors, adenosine regulating agents, cannabinoid derivatives, Substance P antagonists, neurokinin-1 receptor antagonists and sodium channel blockers, among others. Preferred combination therapies comprise use of a composition of the invention with one or more compounds selected from aceclofenac, acemetacin, *e*-acetamidocaproic acid, acetaminophen, acetaminosalol, acetanilide, acetylsalicylic acid (aspirin), S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5 -nitro-2 -propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalinide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen and zomepirac (see The Merck Index, 12th Edition (1996), Therapeutic Category and Biological Activity Index, lists therein headed "Analgesic", "Anti-inflammatory" and "Antipyretic").

Particularly preferred combination therapies comprise use of a composition of the invention with an opioid compound, more particularly where the opioid compound is codeine, meperidine, morphine or a derivative thereof.

The compound to be administered in combination with valdecoxib can be formulated separately from the drug or co-formulated with the drug in a composition of the invention. As a reference example, where celecoxib is co-formulated with a second drug, for example an opioid drug, the second drug can be formulated in immediate-release, rapid-onset, sustained-release or dual-release form.

In an embodiment of the invention, particularly where the cyclooxygenase-2 mediated condition is headache or migraine, the valdecoxib composition is administered in combination therapy with a vasomodulator, preferably a xanthine derivative having vasomodulatory effect, more preferably an alkylxanthine compound.

Combination therapies wherein an alkylxanthine compound is co-administered with a valdecoxib composition as provided herein are embraced by the present embodiment of the invention whether or not the alkylxanthine is a vasomodulator and whether or not the therapeutic effectiveness of the combination is to any degree attributable to a vasomodulatory effect. The term "alkylxanthine" herein embraces xanthine derivatives having one or more C₁₋₄ alkyl, preferably methyl, substituents, and pharmaceutically acceptable salts of such xanthine derivatives. Dimethylxanthines and trimethylxanthines, including caffeine, theobromine and theophylline, are especially preferred. Most preferably, the alkylxanthine compound is caffeine.

The total and relative dosage amounts of valdecoxib and of the vasomodulator or alkylxanthine are selected to be therapeutically and/or prophylactically effective for relief of pain associated with the headache or migraine. Suitable dosage amounts will depend on the particular vasomodulator or alkylxanthine selected. As a reference example, in a combination therapy with celecoxib and caffeine, typically the celecoxib will be administered in a daily dosage amount of about 50 mg to about 1000 mg, preferably about 100 mg to about 600 mg, and the caffeine in a daily dosage amount of about 1 mg to about 500 mg, preferably about 10 mg to about 400 mg, more preferably about 20 mg to about 300 mg.

The vasomodulator or alkylxanthine component of the combination therapy can be administered in any suitable dosage form by any suitable route, preferably orally. The vasomodulator or alkylxanthine can optionally be coformulated with the selective cyclooxygenase-2 inhibitory drug in the molded article of the invention. Thus a molded article of the invention optionally comprises both an aminosulfonyl-comprising valdecoxib and a vasomodulator or alkylxanthine such as caffeine, in total and relative amounts consistent with the dosage amounts set out hereinabove.

The phrase "in total and relative amounts effective to relieve pain", with respect to amounts of valdecoxib and a vasomodulator or alkylxanthine in a composition of the present embodiment, means that these amounts are such that (a) together these components are effective to relieve pain, and (b) each component is or would be capable of contribution to a pain-relieving effect if the other component is or were not present in so great an amount as to obviate such contribution.

Excipient ingredients forming the carrier system for the selective cyclooxygenase-2 inhibitory drug in a molded article of the invention include at least one pharmaceutically acceptable carbohydrate. The carbohydrate(s) can function as bulking agents, as swelling agents, as wicking agents, as binders and/or in other ways. Illustratively, the carbohydrate(s) can be selected from natural and modified celluloses, *e*.*g*., microcrystalline cellulose, methylcellulose, ethylcellulose, hydroxypropylcellulose, hydroxypropyl methyleellulose, carboxymethylcellulose, or sodium carboxymethylcellulose, natural and modified starches, *e*.*g*., corn starch, pregelatinized starch, sodium starch glycolate, and mono-, di- and oligosaccharides having up to 6 saccharide units, including sugars and sugar alcohols, *e*.*g*., erythritol, glucose, lactose, maltitol, maltose, mannitol, sorbitol, sucrose, xylitol.

It is preferred that at least one carbohydrate substantially present in the carrier system is selected from sugars and sugar alcohols, more preferably those exhibiting rapid dissolution in the mouth, most preferably those exhibiting such rapid dissolution and providing a sweet taste. Sugars and sugar alcohols having high moldability, *e*.*g*., maltitol, maltose and sorbitol, as well as sugars and sugar alcohols having low moldability, particularly when in finely particulate as opposed to granular form, e.g., glucose, lactose, mannitol, sucrose and xylitol, can be useful.

Selection of suitable carbohydrates can readily be made by reference to the above-cited patents describing processes for preparing oral fast-melt pharmaceutical formulations.

One or more carbohydrates are present in the molded article of the invention in a total amount of about 20% to about 60%, and preferably about 25% to about 50%, for example about 40%, by weight of the molded article.

Optionally, a molded article of the invention can contain one or more additional pharmaceutically acceptable excipients including, but not limited to, binders, wetting agents, water-soluble lubricants, water-insoluble lubricants, disintegrants, glidants, sweeteners, flavoring agents, effervescent agents, colorants. Such optional additional components should be physically and chemically compatible with the other ingredients of the molded article and must not be deleterious to the recipient. Selection of suitable excipients can readily be made by reference to the above-cited patents describing processes for preparing oral fast-melt pharmaceutical formulations. Some of the excipients mentioned illustratively below are carbohydrates and are therefore already included in the category of carbohydrates described above.

Pharmaceutically acceptable binders that can optionally be present in a molded article of the invention include, individually or in combination, gums, polypeptides, natural and modified starches, cellulosic materials, alginic acid and salts thereof, polyethylene glycol, polyvinylpyrrolidone, polymethacrylates, silicate salts and bentonites.

Preferred gums include acacia, carrageenan, guar, locust bean, karaya, tragacanth and xanthan gums. A preferred polypeptide is gelatin. Preferred starches include corn starch and pregelatinized starch. Preferred cellulosic materials include microcrystalline cellulose, methylcellulose, ethylcellulose, hydroxypropylcellulose, hydroxypropylmethylcellulose, carboxymethylcellulose and salts thereof, e.g., sodium carboxymethylcellulose. A preferred alginic acid salt is sodium alginate. A preferred silicate salt is magnesium aluminum silicate.

One or more binding agents, if desired, are present in a total amount of about 1% to about 10%, preferably about 1% to about 7.5%, and more preferably about 1% to about 5%, by weight of the molded article.

Pharmaceutically acceptable wetting agents that can optionally be present in a molded article of the invention include, individually or in combination, surfactants, hydrophilic polymers and certain clays. Wetting agents can be useful to aid in wetting of a hydrophobic drug, such as celecoxib, during the formulation process.

Non-limiting examples of surfactants that can be useful include quaternary ammonium compounds, e.g., benzalkonium chloride, benzethonium chloride and cetylpyridinium chloride, dioctyl sodium sulfosuccinate, polyoxyethylene alkylphenyl ethers, e.g., nonoxynol 9, nonoxynol 10, and octoxynol 9, poloxamers (polyoxyethylene and polyoxypropylene block copolymers), polyoxyethylene fatty acid glycerides and oils, e.g., polyoxyethylene (8) caprylic/capric mono- and diglycerides, polyoxyethylene (35) castor oil and polyoxyethylene (40) hydrogenated castor oil, polyoxyethylene alkyl ethers, e.g., polyoxyethylene (20) cetostearyl ether, polyoxyethylene fatty acid esters, e.g., polyoxyethylene (40) stearate, polyoxyethylene sorbitan esters, e.g., polysorbate 20 and polysorbate 80, propylene glycol fatty acid esters, e.g., propylene glycol laurate, sodium lauryl sulfate, fatty acids and salts thereof, e.g., oleic acid, sodium oleate and triethanolamine oleate, glyceryl fatty acid esters, e.g., glyceryl monostearate, sorbitan esters, e.g., sorbitan monolaurate, sorbitan monooleate, sorbitan monopalmitate and sorbitan monostearate, tyloxapol, and mixtures thereof. Sodium lauryl sulfate is a preferred wetting agent in molded articles of the invention.

One or more wetting agents, if desired, are present in a molded article of the invention in a total amount of about 0.05% to about 5%, preferably about 0.075% to about 2.5%, and more preferably about 0.25% to about 1%, for example about 0.5%, by weight of the molded article.

Pharmaceutically acceptable water-insoluble lubricants that can optionally be present in a molded article of the invention include glyceryl behapate, stearates (e.g., magnesium, calcium and sodium stearates), stearic acid, hydrogenated vegetable oils, colloidal silica, talc, waxes and mixtures thereof. Optionally a water-insoluble lubricant can be used in mixture with a wetting agent, as for example in calcium stearate/sodium lauryl sulfate mixtures (*e.g.*, Sterowet™).

Magnesium stearate, stearic acid and mixtures thereof are preferred water-insoluble lubricants.

One or more water-insoluble lubricants, if desired, are present in a molded article of the invention in a total amount of about 0.05% to about 5%, preferably about 0.75% to about 2.5%, and more preferably about 1% to about 2%, for example about 1.5%, by weight of the molded article.

Pharmaceutically acceptable water-soluble lubricants that can optionally be present in a molded article of the invention include boric acid, sodium benzoate, sodium acetate, sodium fumarate, sodium chloride, DL-leucine, polyethylene glycols (*e*.*g*., Carbowax™ 4000 and Carbowax™ 6000), sodium oleate and mixtures thereof.

One or more water-soluble lubricants, if desired, are present in a molded article of the invention in a total amount of about 0.05% to about 5%, preferably about 0.75% to about 2.5%, and more preferably about 1% to about 2%, for example about 1.5%, by weight of the molded article.

Pharmaceutically acceptable disintegrants that can optionally be present in a molded article of the invention include starches, sodium starch glycolate, clays, *e*.*g*., Veegum™ HV, celluloses, *e*.*g*., purified cellulose, methylcellulose, sodium carboxymethylcellulose carboxymethylcellulose, croscarmellose sodium, alginates, pregelatinized corn starches, *e*.*g*., National™ 1551 and National™ 1550, crospovidone, gums, *e*.*g*., agar, guar, locust bean, karaya, pectin and tragacanth gums, and mixtures thereof. Croscarmellose sodium and sodium starch glycolate are preferred disintegrants.

One or more disintegrants, if desired, are present in a molded article of the invention in a total amount of about 0.5% to about 7.5%, preferably about 1% to about 5%, and more preferably about 1% to about 3.5%, by weight of the molded article.

Optionally, an effervescent salt can be used as a disintegrant and to enhance organoleptic properties of a fast-melt tablet of the invention.

Pharmaceutically acceptable glidants that can optionally be present in a molded article of the invention, for example to enhance flow of tableting material into tablet dies, to prevent sticking of tableting material to punches and dies, or to produce tablets having a sheen, include silicon dioxide products such as fumed silica (e.g., Cab-O-Sil™ of Cabot Corp. and Aerosil™ of Degussa). Silicon dioxide, if desired, is present in a molded article of the invention in a total amount of about 0.05% to about 5%, preferably about 0.1 % to about 2%, and more preferably about 0.25% to about 1 %, for example about 0.5%, by weight of the molded article.

Pharmaceutically acceptable sweeteners that can optionally be present in a molded article of the invention in a sweetening effective amount include mannitol, propylene glycol, sodium saccharin, acesulfame K, neotame, and aspartame.

Pharmaceutically acceptable flavoring agents that can optionally be present in a molded article of the invention in a flavoring effective amount include peppermint, spearmint, grape, cherry, strawberry, and lemon.

The molded article of the invention can take any suitable form, including a wafer, a lozenge or a tablet. Optionally, the molded article can be scored or otherwise provided with means for convenient breaking into unit-dose segments, but preferably the molded article is a self-contained dosage form delivering a single unit dose. In a preferred embodiment, the molded article is an oral fast-melt tablet.

Tablets of the invention can be made to any desired size, for example 8 mm, 10 mm, 12 mm, shape, for example round, oval, oblong, weight, and thickness. Optionally, tablets of the invention can have etchings or monograms on one or both sides.

Tablets of the invention disintegrate within 30 to 300 seconds, preferably within 30 to 200 seconds, and more preferably within 30 to 150 seconds, in a standard *in vitro* disintegration assay (e.g., conducted according to U.S. Pharmacopeia 24 (2000), Test No. 701).

Additionally, preferred tablets of the invention disintegrate within about 5 to about 60 seconds, more preferably within about 5 to about 40 seconds, and still more preferably within about 5 to about 30 seconds, for example within about 25 seconds, after placement in the oral cavity of a subject.

Tablets of the invention have a hardness that can depend on size and shape as well as on composition, among other characteristics. Tablet hardness can be measured by any method known in the art, for example by a tablet hardness meter (e.g., Schleuniger). Preferably, compositions of the invention have a hardness of about 9.8 N to about 98 N (about 1 to about 10 kp), and more preferably of about 9.8 N to about 49 N (about 1 to about 5 kp).

In a presently preferred embodiment, solid dosage forms of the invention have sufficient hardness for handling and, therefore, can be put into practical use in the same manner as the case of swallowable tablets. The term "sufficient hardness for handling" as used herein means a hardness which can withstand removal from at least a standard type of blister packaging, or such a hardness as will withstand other handling such as packaging, delivery, carrying and the like.

Tablets of the invention preferably have a minimum hardness so as to resist breakage of the tablet during removal from standard blister packaging by pushing the tablet through a cover sheet. A suitable hardness is about 9.8 N (1 kp) or more for a tablet having a diameter of about 8 mm, about 14.7 N (1.5 kp) or more for a tablet having a diameter of about 10 mm, and about 19.6 N (2 kp) or more when the tablet has a diameter of about 12 mm.

In another presently preferred embodiment, tablets of the invention have sufficient hardness such that a plurality of such tablets can be packaged together, for example in a glass or plastic bottle, without individual packaging, yet do not exhibit substantial breakage or sticking and/or melding together during normal shipping and handling. Tablets intended for such packaging preferably have a hardness of about 3 kp or more.

Tablets of the invention can be packaged in any suitable manner known in the art. A multiplicity of fast-melt tablets can be packaged together, for example in a glass or plastic bottle or container. Alternatively, fast-melt tablets of the invention can be individually wrapped, for example in plastic or foil, or packaged in known forms of blister packaging. Blister packaging with improved force distribution properties such as is disclosed in U.S. Patent No. 5,954,204 to Grabowski can be especially useful to package fast-melt tablets of the invention.

Tablets of the invention can be taken by a subject by any oral administration means in accordance with the subject's choice or condition. For example, fast-melt tablets can be taken without water. Upon placement in the oral cavity and especially in the cheek or above the tongue, such a tablet is exposed to saliva and rapidly disintegrates therein. The rate of disintegration increases further when an intraoral pressure, for example a pressure between the palate and tongue or a licking or sucking pressure, is applied to the tablet.

Alternatively, a tablet of the invention can be taken with the aid of water in an amount sufficient to wet the oral cavity and to assist in disintegration of the tablet. Also, a tablet can be swallowed together with a small amount of water after complete or partial disintegration in the oral cavity. Tablets of the invention can also be swallowed directly with water.

Numerous processes not involving wet granulation but suitable for preparing a molded article of the invention are available. In one embodiment, the molded article is prepared by a process comprising a direct compression step. In another embodiment, the process comprises a step following the shaping step wherein a solvent, preferably an aqueous solvent, is removed from the molded article by freeze-drying, vacuum-drying or lyophilization. In another embodiment, the molded article is prepared by a process wherein the excipient carrier system is prepared as a shearform matrix (i.e., a fibrous mass similar to cotton candy) to which the drug is added, and the shaping step comprises compression of the shearform matrix to form the molded article. As part of any process contemplated herein, the drug can, if desired, be coated with a taste-masking composition.

Illustrative examples of these and other processes for preparing a molded article of the invention are presented with greater particularity below.

In one particular embodiment of the invention, the molded article is a porous tablet prepared by providing a mix comprising an inert readily volatilizable solid adjuvant, for example urethane, urea, ammonium carbonate, ammonium bicarbonate, hexamethylenetetramine, benzoic acid, phthalic anhydride, naphthalene, or camphor, compressing the mix to form a tablet; and thereafter volatilizing the adjuvant to form a porous tablet substantially as disclosed in above-cited U.S. Patent No. 3,885,026. The selective cyclooxygenase-2 inhibitory drug and other desired excipients are present in the mix.

In another particular embodiment of the invention, the molded article is a porous tablet prepared by forming a mixture of the tablet components with a solvent, for example water, or cyclohexane, which is inert towards the tablet components and which has a freezing point of about -30°C to about 25°C, the solvent constituting about 5% to about 80% by weight of the mixture; solidifying the mixture by introduction into an inert cooling medium; compressing the mixture at a temperature below the freezing point of the solvent to form a tablet; and thereafter evaporating the solvent to form a porous tablet substantially as disclosed in above-cited U.S. Patent No. 4,134,943. A selective cyclooxygenase-2 inhibitory drug and other desired excipients are present in the mix.

In another particular embodiment of the invention, the molded article is a tablet formed by placing in a mold a composition comprising or consisting essentially of the drug and a solution of a water-soluble or water-dispersible carrier material, for example a polypeptide such as partially hydrolyzed gelatin or a polysaccharide such as hydrolyzed dextran, dextrin, or sodium alginate, alone or in mixture with other carrier materials such as polyvinyl alcohol, polyvinylpyrrolidone, or acacia, in a solvent, preferably water; and subliming, for example by freeze-drying, the solvent to form a tablet within the mold substantially as disclosed in above-cited U.S. Patent No. 4,371,516. The mold can be a depression in a filmic material suitable as packaging material for the tablet, and a peelable cover sheet can thereafter be adhered to the filmic material, thereby covering the tablet, substantially as disclosed in above-cited U.S. Patent No. 4,305,502.

In a related embodiment, the process comprises suspending the drug in a melted triglyceride vehicle; spray-congealing the resulting suspension to form discrete solid particles having the drug encapsulated therein; mixing the drug-containing particles with a water-soluble but ethanol-insoluble carbohydrate, for example fructose, dextrose, lactose, or sucrose, and a solvent, for example a water-ethanol mixture, to form a damp mass; compressing the damp mass in a mold to form a tablet; and removing the solvent by drying, substantially as disclosed in above-cited U.S. Patent No. 5,082,667.

In another particular embodiment of the invention, the molded article is a rapidly water-disintegratable tablet comprising the drug, and having distributed therewithin a small but effective amount of a tablet disintegrating system comprising an unreacted, intimate mixture of alginic acid and a water-soluble metal carbonate in proportions reactive to form alginic acid salt and carbonic acid when the tablet is placed in water, substantially as disclosed in above-cited U.S. Patent No. 4,414,198.

In another particular embodiment of the invention, the molded article comprises a mass of spun fibers of a readily water-soluble material, for example a sugar such as sucrose, fructose, dextrose, mannitol, sorbitol, lactose, or maltose, or a cellulosic material such as methylcellulose, ethylcellulose, hydroxymethylcellulose, hydroxyethylcellulose, or alkali metal salts of carboxymethylcellulose, having the drug distributed on or incorporated in the mass of spun fibers, substantially as disclosed in above-cited U.S. Patent No. 4,855,326. The mass of spun fibers can be formed as in a process for producing cotton candy. This is a melt extrusion process wherein a stock material comprising the water-soluble material, having the selective cyclooxygenase-2 inhibitory drug dispersed therein, is melted and forced through spinnerets. The resulting cotton candy-like material can be lightly compacted to form the molded article of the invention.

In a related embodiment, the molded article is prepared by a process comprising preparing a shearform matrix, for example by a flash-flow process, from a feedstock comprising a saccharide component, for example sucrose optionally mixed with other saccharides such as dextrose, sorbitol, or mannitol, optionally with a crystallization enhancer such as a surfactant; adding a crystallization/binding promoter such as an alcohol, e.g., ethanol, polyvinylpyrrolidone or a mixture thereof, and the drug to the shearform matrix to at least partially crystallize the shearform matrix; and thereafter compacting the shearform matrix to form a molded article substantially as disclosed in above-cited U.S. Patents No. 5,587,172 and No. 5,869,098.

In a further related embodiment, the molded article is prepared by a process comprising mixing the drug with a shearform matrix, compacting the resulting mixture in a mold, and curing the compacted mixture by subjecting to environmental conditions of heat, moisture and pressure that induce crystallization to form a molded article substantially as disclosed in above-cited U.S. Patent No. 5,622,719.

In any embodiment of the invention having a shearform matrix or matrix of spun fibers, the drug can optionally be formulated using known controlled-release, delayed-release or sustained-release delivery systems, substantially as disclosed in above-cited U.S. Patent No. 5,733,577.

Suitable apparatus for preparing molded articles from a shearform matrix has been disclosed, for example in above-cited U.S. Patents No. 5,653,926 and No. 5,662,849.

In another particular embodiment of the invention, the molded article comprises an open matrix network having the drug distributed therein, the open matrix network being formed from mannitol in admixture with a gum, for example acacia, guar gum, xanthan gum, tragacanth gum, locust bean gum, pectin, algin, agar, carrageenan, or gum arabic, substantially as disclosed in above-cited U.S. Patent No. 4,946,684.

In another particular embodiment of the invention, the molded article is a tablet comprising a directly compressible solid excipient, typically a sugar, for example sucrose, lactose or sorbitol; a lubricant, preferably a water-soluble lubricant such as sodium dodecyl sulfate; and the drug; and is prepared by mixing the ingredients to form a mixture and directly compressing the mixture to form a tablet substantially as disclosed in above-cited U.S. Patent No. 5,073,374.

In another particular embodiment of the invention, the molded article is a tablet comprising a water- or saliva-activated effervescent disintegration agent and microparticles containing the drug. The drug can optionally be taste-masked by substantially encompassing it with a protective material in the microparticles, substantially as disclosed in above-cited U.S. Patent No. 5,178,878. In a related embodiment, a particulate effervescent couple is intimately mixed with the drug, each particle of the effervescent couple comprising a solid core of an edible acid and a coating of an edible base in amounts such that upon reaction of the acid and the base a portion of free unreacted acid remains, substantially as disclosed in above-cited U.S. Patent No. 5,503,846.

In another particular embodiment of the invention, the molded article is a tablet formed by preparing a mixture comprising the drug and a matrix that comprises a gum, for example acacia, guar gum, xanthan gum, or tragacanth gum, a carbohydrate base, for example mannitol, dextrose, sucrose, lactose, maltose, maltodextrin, or corn syrup solids, and a solvent; shaping the mixture to form a tablet; freezing the mixture; and vacuum drying the frozen mixture above the collapse temperature of the mixture to form a partially collapsed matrix network substantially as disclosed in above-cited U.S. Patent No. 5,298,261. The "collapse temperature" is the initial melting point or eutectic temperature of the matrix.

In another particular embodiment of the invention, the molded article is a compact tablet at least 50% by weight of which is the drug, and having as inactive ingredients at least one cellulose and/or cellulose derivative, at least one soluble sugar alcohol, at least one sweetener and at least one flavoring agent, substantially as disclosed in above-cited U.S. Patent No. 5,401,514 but with replacement of the water-soluble expectorant disclosed therein by a selective cyclooxygenase-2 inhibitory drug, preferably one of low water solubility.

In another particular embodiment of the invention, the molded article is a tablet comprising the drug in a form of coated or non-coated microcrystals or microgranules, and a mixture of excipients comprising a disintegrating agent, preferably a carboxymethylcellulose or an insoluble reticulated polyvinylpyrrolidone, a swelling agent, preferably a starch, a modified starch such as a carboxymethylated starch or a microcrystalline cellulose, and optionally a direct compression sugar such as dextrose, substantially as disclosed in above-cited U.S. Patent No. 5,464,632.

In another particular embodiment of the invention, the molded article is prepared by a process comprising suspending the drug and a sugar comprising lactose and/or mannitol in a 0.3% to 2% by weight aqueous solution of agar used in an amount of 40% to 60% by weight based on the solid components to form a suspension; filling the suspension into a mold and permitting it to set therein to form a jelly; and thereafter drying the jelly to form the molded article, substantially as disclosed in above-cited U.S. Patent No. 5,466,464.

In another particular embodiment of the invention, the molded article is a tablet prepared by a process comprising mixing the drug, a carbohydrate, for example sucrose, starch sugars, sugar alcohols, or tetroses, and a barely sufficient amount of water to wet the surface of particles of the carbohydrate, to form a compressible composition, and compression molding the composition to form a tablet substantially as disclosed in above-cited U.S. Patent No. 5,501,861.

In another particular embodiment of the invention, the molded article is a tablet prepared by a process comprising providing an aqueous composition that comprises (a) an aqueous medium, (b) a support agent comprising a polymeric component, for example a non-hydrolyzed gelatin, capable of maintaining a net charge, a solubilizing component, for example a hydrolyzed gelatin, more water-soluble than the polymeric component and capable of maintaining a net charge of the same sign as the polymeric component, and a bulking agent, (c) a volatilizing agent, for example an alcohol, and (d) a buffering agent; drying the aqueous composition, for example by spray drying, to form a particulate support matrix; adding the drug to the particulate support matrix; and compacting the resulting mixture to form a tablet substantially as disclosed in above-cited U.S. Patents No. 5,587,180 and No. 5,807,576.

In another particular embodiment of the invention, the molded article comprises an orally disintegrating delivery system, for example an effervescent delivery system, having incorporated therein microparticles each having a core comprising the drug and a compound which is sweet in taste and has a negative heat of solution, for example mannitol, and a coating comprising a film-forming polymer such as ethylcellulose, substantially as disclosed in above-cited U.S. Patent No. 5,607,697. This embodiment is especially useful where enhanced taste-masking is desired.

In another particular embodiment of the invention, the molded article is a tablet prepared by a process comprising adding a volatile salt to the drug, a binder such as trehalose, particularly anhydrous trehalose, an additional binder, and other optional excipients with mixing to form a substantially homogeneous mixture; and compressing the mixture to form a tablet substantially as disclosed in above-cited U.S. Patent No. 5,762,961.

In another particular embodiment of the invention, the molded article is a tablet prepared by kneading a mixture of the drug and a readily water-soluble crystalline or powdery solid, preferably one having a sweet taste such as sucrose, lactose, glucose, fructose, xylitol, sorbitol, or mannitol, with a suitable amount of water, typically about 1% to about 10% by weight of the tablet components; compressively shaping the resulting wet kneaded mixture to form a tablet; and thereafter drying the tablet substantially as disclosed in above-cited U.S. Patent No. 5,837,285.

In another particular embodiment of the invention, the molded article is a tablet or wafer prepared by a process comprising coating particles of the drug with a taste-masking composition, preferably one that comprises a first polymer selected from cellulose acetate and cellulose acetate butyrate and a second polymer selected from polyvinylpyrrolidone and hydroxypropylcellulose in a weight ratio of first polymer to second polymer of about 90:10 to about 50:50; dry-blending the coated drug particles with a compressible carbohydrate, for example mannitol, sorbitol, dextrose, sucrose, xylitol, or lactose, and a binder, for example cellulose (in particular microcrystalline cellulose), cellulosic derivatives, polyvinylpyrrolidone, starch, or modified starch; and the resulting dry blend is compressed to form a tablet or wafer substantially as disclosed in above-cited U.S. Patent No. 5,876,759.

In another particular embodiment of the invention, the molded article is a tablet prepared by a process comprising a step of preparing compact granules by pre-compacting by mechanical means, for example rolling, or by spray-drying a feedstock comprising a carbohydrate, for example a saccharide of low or high moldability such as maltose, maltitol, sorbitol, mannitol, glucose, sucrose, or xylitol, and optionally a low density alkaline-earth metal salt; and a step of compressing the compact granules, optionally with other ingredients, to prepare the tablet substantially as disclosed in above-cited U.S. Patent No. 5,939,091. A selective cyclooxygenase-2 inhibitory drug can be added at any stage in the process to result in a molded article of the present invention.

In another particular embodiment of the invention, the molded article is a tablet prepared by a process comprising mixing the drug and a carrier that comprises one or more carbohydrates and a binder to form a blend; kneading the blend with about 1% to about 10% by weight of water; drying the kneaded blend and milling to form a compressible powder; and compressing the powder to form a tablet. The carbohydrates can include saccharides and starches, for example erythritol and microcrystalline cellulose substantially as disclosed in above-cited U.S. Patent No. 5,958,453.

In another particular embodiment of the invention, the molded article is a tablet prepared by freeze-drying and comprising the drug, a matrix forming agent such as a maltodextrin having a dextrose equivalent value of about 12 to about 40 or isomalt, and a binding agent, substantially as disclosed in above-cited U.S. Patent No. 6,010,719.

In another particular embodiment of the invention, the molded article is a tablet prepared by direct compression and comprising the drug, a non-direct compression filler, preferably a non-direct compression sugar or sugar alcohol such as dextrose, mannitol, sorbitol, lactose, or sucrose, and a lubricant, substantially as disclosed in above-cited U.S. Patent No. 6,024,981.

## Claims

1. A molded article for administration to an oral cavity of a subject to treat or prevent a cyclooxygenase-2 mediated condition, disorder or disease, the molded article comprising a moldable blend of a therapeutically effective amount of the selective cyclooxygenase-2 inhibitory drug valdecoxib with a pharmaceutically acceptable excipient carrier system comprising one or more carbohydrates, wherein said carbohydrates constitute more than 50% by weight of all excipients in the moldable blend, wherein ingredients and amounts thereof in the molded article and a process for preparing the molded article are selected such that the molded article exhibits rapid disintegration in the oral cavity, and wherein the moldable blend is prepared by a process step not requiring wet granulation, wherein said article disintegrates within 30 to 300 seconds after placement in a standard in vitro disintegration assay, conducted according to U.S. Pharmacopeia 24 (2000), Test No. 701, and wherein the selective cyclooxygenase-2 inhibitory drug is present in a total amount of 20% to 60% by weight of the molded article.

2. The molded article of Claim 1 wherein the carbohydrate(s) present in the excipient carrier system are selected from natural and modified celluloses, natural and modified starches, mono-, di- and oligosaccharide sugars and sugar alcohols.

3. The molded article of Claim 1 wherein at least one carbohydrate present in the excipient carrier system is a sugar or sugar alcohol.

4. The molded article of Claim 3 wherein the sugar or sugar alcohol is selected from erythritol, glucose, lactose, maltitol, maltose, mannitol, sorbitol, sucrose and xylitol.

5. The molded article of Claim 3 wherein the sugar or sugar alcohol is one that exhibits rapid dissolution in the oral cavity of a subject and provides a sweet taste.

6. The molded article of Claim 1 wherein one or more carbohydrates are present in a total amount of 20% to 60% by weight of the molded article.

7. The molded article of Claim 1 that is an oral fast-melt tablet.

8. The tablet of Claim 7 that disintegrates within 5 to 60 seconds after placement in the oral cavity of a subject.

9. The tablet of Claim 7 having a hardness of 9.8 to 98 N (1 to 10 kp).

10. The tablet of Claim 7 having sufficient hardness to resist breakage of the tablet during removal from standard blister packaging by pushing the tablet through a cover sheet, wherein the hardness is 9.8 N (1 kp) or more for a tablet having a diameter of about 8 mm, 14.7 N (1.5 kp) or more for a tablet having a diameter of about 10 mm, and 19.6 N (2 kp) or more when the tablet has a diameter of about 12 mm.

11. The tablet of Claim 7 having sufficient hardness to enable tablets to be packaged together in a glass or plastic bottle, without individual packaging, whereby the tablets do not exhibit substantial breakage or sticking and/or melding together during normal shipping and handling, wherein the hardness is 29.4 N (3 kp) or more.

12. A process for preparing a molded article which is an oral fast-melt dosage form of the selective cyclooxygenase-2 inhibitory drug valdecoxib, the process comprising a step of intimately mixing valdecoxib in a therapeutically effective amount with an excipient carrier system comprising one or more carbohydrates, to form a blend, wherein formation of the blend does not require wet granulation and wherein said carbohydrates constitute more than 50% by weight of said blend; and a step of shaping a unit-dose quantity of the blend in a mold to form the molded article; wherein said article disintegrates within 30 to 300 seconds after placement in a standard in vitro disintegration assay, conducted according to U.S. Pharmacopeia 24 (2000), Test No. 701, and wherein the selective cyclooxygenase-2 inhibitory drug is present in a total amount of 20% to 60% by weight of the molded article.

13. The process of Claim 12 wherein the carbohydrate(s) present in the excipient cartier system are selected from natural and modified celluloses, natural and modified starches, mono-, di- and oligosaccharide sugars and sugar alcohols.

14. The process of Claim 12 wherein at least one carbohydrate present in the excipient carrier system is a sugar or sugar alcohol.

15. The process of Claim 14 wherein the sugar or sugar alcohol is selected from erythritol, glucose, lactose, maltitol, maltose, mannitol, sorbitol, sucrose and xylitol.

16. The process of Claim 14 wherein the sugar or sugar alcohol is one that exhibits rapid dissolution in the oral cavity of a subject and provides a sweet taste.

17. The process of Claim 12 wherein one or more carbohydrates are present in a total amount of 20% to 60% by weight of the molded article.

18. The process of Claim 12 wherein the shaping step comprises direct compression of the blend to form a tablet.

19. The process of Claim 12 further comprising a step of removing a solvent from the molded article by freeze-drying, vacuum-drying or lyophilization.

20. The process of Claim 12 wherein the excipient carrier system is prepared as a shearform matrix to which the drug is added, and wherein the shaping step comprises compression of the shearform matrix.

21. Use of a molded article of Claim 1 for the manufacture of a medicament for treating a medical condition or disorder in a mammalian subject where treatment with a cyclooxygenase-2 inhibitor is indicated, wherein the medicament is adapted for oral administration, and wherein the medical condition is selected from the group consisting of inflammation, pain, fever, arthritis, arthritic disorders, rheumatoid arthritis, spondyloarthropathies, gouty arthritis, osteoarthritis, systemic lupus erythematosus, juvenile arthritis, asthma, bronchitis, menstrual cramps, preterm labor, tendinitis, bursitis, allergic neuritis, cytomegalovirus infectivity, apoptosis including HIV-induced apoptosis, lumbago, liver disease including hepatitis, psoriasis, eczema, acne, bums, dermatitis, ultraviolet radiation damage including sunburn, post-operative inflammation including that following ophthalmic surgery such as cataract surgery or refractive surgery; gastrointestinal conditions including inflammatory bowel disease, Crohn's disease, gastritis, irritable bowel syndrome, ulcerative colitis; inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia; ophthalmic diseases, such as retinitis, scleritis, episcleritis, conjunctivitis, retinopathies, uveitis, ocular photophobia, acute injury to eye tissue; pulmonary inflammation, such as that associated with viral infections and cystic fibrosis; central nervous system disorders, such as cortical dementias including Alzheimer's disease, neurodegeneration, and central nervous system damage resulting from stroke, ischemia and trauma; dementias, including vascular dementia, multi-infarct dementia, pre-senile dementia, alcoholic dementia and senile dementia; allergic rhinitis, respiratory distress syndrome, endotoxin shock syndrome; pain, including postoperative pain, dental pain, muscular pain, and pain resulting from cancer; pain, fever and inflammation in conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, bums, and trauma following surgical and dental procedures; inflammation-related cardiovascular disorders; vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries; angiogenesis-related disorders, such as tumor angiogenesis; neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and glaucoma, including neovascular glaucoma; ulcerative diseases such as gastric ulcer; hemangiomas, including infantile heznangiomas, angiofibroma of the nasopharynx and avascular necrosis of bone; disorders of the female reproductive system such as endometriosis; benign and malignant tumors/neoplasia including cancers, such as colorectal cancer, brain cancer, bone cancer, epithelial cell-derived neoplasia such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that affect epithelial cells throughout the body; fibrosis that occurs with radiation therapy; adenomatous polyps, including familial adenomatous polyposis; prostanoid-induced smooth muscle contraction, and eosinophil-related disorders; bone loss particularly in postmenopausal women, osteoporosis.

22. The use of Claim 21 wherein said mammalian subject is a human subject.

23. The use of Claim 22 wherein the medicament is adapted for combination therapy with one or more drugs selected from opioids and other analgesics.

24. The use of Claim 22 wherein the medicament is adapted for combination therapy with an opioid compound selected from codeine, meperidine and morphine.

## Patentansprüche

1. Geformter Gegenstand zur Verabreichung in die Mundhöhle eines Subjekts um einen Cyclooxygenase-2-vermittelten Zustand, eine Cyclooxygenase-2-vermittelte Störung oder eine Cyclooxygenase-2-vermittelte Krankheit zu behandeln oder zu verhindern, wobei der geformte Gegenstand eine formbare Mischung aus einer therapeutisch wirksamen Menge des selektiven Cyclooxygenase-2-Inhibitor-Wirkstoffs Valdecoxib mit einem pharmazeutisch annehmbaren Excipiens-Trägersystem, das ein oder mehrere Kohlenhydrate umfasst, wobei die Kohlenhydrate mehr als 50 Gew.-% aller Excipienzien in der formbaren Mischung bilden und wobei die Ingredienzien und die Mengen derselben in dem geformten Gegenstand und ein Verfahren zur Herstellung des geformten Gegenstands so ausgewählt sind, dass der geformte Gegenstand einen schnellen Zerfall in der Mundhöhle aufweist, und wobei die formbare Mischung durch einen Verfahrensschritt hergestellt ist, der keine Nassgranulierung erfordert, wobei der Gegenstand in 30 bis 300 Sekunden nach Platzierung in einem Standard-in-vitro-Zerfallsassay, der entsprechend der US Pharmakopöe 24 (2000), Test Nr. 701, durchgeführt wird, zerfällt und wobei der selektive Cyclooxygenase-2-Inhibitor-Wirkstoff in einer Gesamtmenge von 20 Gew.-% bis 60 Gew.-% des geformten Gegenstands vorliegt.

2. Geformter Gegenstand nach Anspruch 1, wobei das Kohlenhydrat (die Kohlenhydrate), das (die) im Excipiens-Trägersystem vorliegt (vorliegen), aus natürlichen und modifizierten Cellulosen, natürlichen und modifizierten Stärken, Mono-, Di- und Oligosaccharidzuckern und Zuckeralkoholen ausgewählt ist (sind).

3. Geformter Gegenstand nach Anspruch 1, wobei wenigstens ein Kohlenhydrat, dass im Excipiens-Trägersystem vorliegt, ein Zucker oder Zuckeralkohol ist.

4. Geformter Gegenstand nach Anspruch 3, wobei der Zucker oder Zuckeralkohol aus Erythritol, Glucose, Lactose, Maltitol, Maltose, Mannitol, Sorbitol, Saccharose und Xylitol ausgewählt ist.

5. Geformter Gegenstand nach Anspruch 3, wobei der Zucker oder Zuckeralkohol einer ist, der eine schnelle Auflösung in der Mundhöhle eines Subjekts aufweist und einen süßen Geschmack liefert.

6. Geformter Gegenstand nach Anspruch 1, wobei ein Kohlenhydrat oder mehrere Kohlenhydrate in einer Gesamtmenge von 20 Gew.-% bis 60 Gew.-% des geformten Gegenstands vorliegt (vorliegen).

7. Geformter Gegenstand nach Anspruch 1, der eine orale, schnell schmelzende Tablette ist.

8. Tablette nach Anspruch 7, die innerhalb von 5 bis 60 Sekunden nach Platzieren in der Mundhöhle eines Subjekts zerfällt.

9. Tablette nach Anspruch 7, die eine Härte von 9,8 bis 98 N (1 bis 10 kp) hat.

10. Tablette nach Anspruch 7, die eine ausreichende Härte hat, um sich einem Brechen der Tablette während einer Entfernung aus einer Standardblisterverpackung durch Drücken der Tablette durch eine Deckfolie zu widersetzen, wobei die Härte 9,8 N (1 kp) oder mehr für eine Tablette mit einem Durchmesser von etwa 8 mm, 14,7 N (1,5 kp) oder mehr für eine Tablette mit einem Durchmesser von etwa 10 mm und 19,6 N (2 kp) oder mehr ist, wenn die Tablette einen Durchmesser von etwa 12 mm hat.

11. Tablette nach Anspruch 7, die eine ausreichende Härte hat, um es zu ermöglichen, dass Tabletten zusammen in einer Glas- oder Kunststoffflasche ohne einzelne Verpackung verpackt werden, wodurch die Tabletten kein wesentliches Brechen oder Kleben und/oder Verschmelzen miteinander während des normalen Transports und der normalen Handhabung aufweisen, wobei die Härte 29,4 N (3 kp) oder mehr ist.

12. Verfahren zur Herstellung eines geformten Gegenstands, der eine orale, schnell schmelzende Dosierungsform des selektiven Cyclooxygenase-2-Inhibitor-Wirkstoffs Valdecoxib ist, wobei das Verfahren umfasst: einen Schritt des innigen Vermischens von Valdecoxib in einer therapeutisch wirksamen Menge mit einem Excipiens-Trägersystem, dass ein Kohlenhydrat oder mehrere Kohlenhydrate umfasst, unter Bildung einer Mischung, wobei die Bildung der Mischung keine Nassgranulierung erfordert und wobei die Kohlenhydrate mehr als 50 Gew.-% der Mischung bilden; und einen Schritt des Formens einer Einzeldosismenge der Mischung in einer Form unter Bildung des geformten Gegenstands; wobei der Gegenstand innerhalb von 30 bis 300 Sekunden nach Platzierung in einem Standard-in-vitro-Zerfallsassay der entsprechend der US Pharmakopöe 24 (2000), Test Nr. 701, durchgeführt wird, zerfällt und wobei der selektive Cyclooxygenase-2-Inhibitor-Wirkstoff in einer Gesamtmenge von 20 Gew.-% bis 60 Gew.-% des geformten Gegenstands vorliegt.

13. Verfahren nach Anspruch 12, wobei das Kohlenhydrat (die Kohlenhydrate), das (die) im Excipiens-Trägersystem vorliegen, aus natürlichen und modifizierten Cellulosen, natürlichen und modifizierten Stärken, Mono-, Di- und Oligosaccharidzuckern und Zuckeralkoholen ausgewählt ist (sind).

14. Verfahren nach Anspruch 12, wobei wenigstens ein Kohlenhydrat, das im Excipiens-Trägersystem vorliegt, ein Zucker oder Zuckeralkohol ist.

15. Verfahren nach Anspruch 14, wobei der Zucker oder Zuckeralkohol aus Erythritol, Glucose, Lactose, Maltitol, Maltose, Mannitol, Sorbitol, Saccharose und Xylitol ausgewählt wird.

16. Verfahren nach Anspruch 14, wobei der Zucker oder Zuckeralkohol einer ist, der in der Mundhöhle eines Subjekts eine schnelle Auflösung aufweist und einen süßen Geschmack liefert.

17. Verfahren nach Anspruch 12, wobei ein Kohlenhydrat oder mehrere Kohlenhydrate in einer Gesamtmenge von 20 Gew.-% bis 60 Gew.-% des geformten Gegenstandes vorliegt (vorliegen).

18. Verfahren nach Anspruch 12, wobei der Formungsschritt ein direktes Verpressen der Mischung unter Bildung einer Tablette umfasst.

19. Verfahren nach Anspruch 12, das außerdem einen Schritt der Entfernung eines Lösungsmittels aus dem geformten Gegenstand durch Gefriertrocknung, Vakuumtrocknung oder Lyophilisierung umfasst.

20. Verfahren nach Anspruch 12, wobei das Excipiens-Trägersystem als eine Scherform-Matrix hergestellt wird, der der Wirkstoff zugesetzt wird, und wobei der Formungsschritt Verpressen der Scherform-Matrix umfasst.

21. Verwendung eines geformten Gegenstands nach Anspruch 1 für die Herstellung eines Medikaments zur Behandlung eines medizinischen Zustands oder einer medizinischen Störung in einem Säugersubjekt, bei dem eine Behandlung mit einem Cyclooxygenase-2-Inhibitor indiziert ist, wobei das Medikament zur oralen Verabreichung angepasst ist und wobei der medizinische Zustand ausgewählt ist, aus der Gruppe bestehend aus Entzündung, Schmerzen, Fieber, Arthritis, arthritischen Störungen, rheumatoider Arthritis, Spondyloarthropathien, Gichtarthritis, Osteoarthritis, systemischem Lupus erythematodes, juveniler Arthritis, Asthma, Bronchitis, Menstruationskrämpfen, Frühgeburt, Tendinitis, Bursitis, allergischer Neuritis, Cytomegalovirus-Infektiösität, Apoptose, einschließlich HIVinduzierter Apoptose, Lumbago, Lebererkrankung, einschließlich Hepatitis, Psoriasis, Ekzemen, Akne, Verbrennungen, Dermatitis, Schädigung durch ultraviolette Strahlung, einschließlich Sonnenbrand, postoperativer Entzündung, einschließlich der nach Augenoperation wie Kataraktoperation oder refraktiver Operation; gastrointestinalen Krankheitsbildern, einschließlich inflammatorischer Darmkrankheit, Crohnscher Krankheit, Gastritis, Reizdarmsyndrom, Colitis ulcerosa; Entzündung bei solchen Krankheiten wie Migräne, Periarteritis nodosa, Thyroiditis, aplastischer Anämie, Hodgkin-Krankheit, Scleroderma, rheumatischem Fieber, Typ I-Diabetes, Nervenendapparat-Krankheit, einschließlich Myasthenia gravis, Krankheit der Substantia alba, einschließlich multipler Sklerose, Sarkoidose, nephrotischem Syndrom, Behcet-Syndrom, Polymyositis, Gingivitis, Nephritis, Hypersensibilität, Schwellung, auftretend nach Verletzung, einschließlich Hirnödem, Myokardischämie; Augenkrankheiten, z.B. Retinitis, Skleritis, Episkleritis, Konjunctivitis, Retinopathien, Uveitis, Augen-Photophobie, akute Verletzung am Augengewebe; Lungenentzündung, z.B. assoziiert mit Virusinfektionen und zystischer Fibrose; Störungen des Zentralnervensystems, z.B. Hirndemenz, einschließlich Alzheimer-Krankheit, Neurodegeneration und Schädigung des Zentralnervensystems als Resultat von Schlaganfall, Ischämie und Trauma; Demenz, einschließlich Gefäßdemenz, Multiinfarktdemenz, präseniler Demenz; Alkoholdemenz und seniler Demenz; allergischer Rhinitis, Atemnotsyndrom, Endotoxinschocksyndrom; Schmerzen, einschließlich postoperativischen Schmerzen, Zahnschmerzen, Muskelschmerzen und Schmerzen, resultierend aus Krebs; Schmerzen, Fieber und Entzündung bei Zuständen, einschließlich rheumatisches Fieber, Influenza und andere virale Infektionen, einschließlich gängige Erkältung, Kreuzschmerzen und Halsschmerzen, Dismenorrhoe, Kopfweh, Zahnweh, Verrenkungen und Verstauchungen, Myositis, Neuralgie, Synovitis, Arthritis, einschließlich rheumatoider Arthritis, degenerativer Gelenkerkrankungen (Osteoarthritis), Gicht und Spondylarthritis, Bursitis, Verbrennungen und Trauma nach chirurgischen und zahnmedizinischen Eingriffen; entzündungsbedingter kardiovaskulärer Störungen; Gefäßkrankheiten, Krankheit der Koronararterien, Aneurysma, Gefäßabstoßung, Arteriosklerose, Atherosklerose, einschließlich Herztransplantatatherosklerose, Myokardinfarkt, Embolie, Schlaganfall, Thrombose, einschließlich Venenthrombose, Angina, einschließlich instabiler Angina, Koronarplaqueentzündung, durch Bakterien induzierte Entzündung, einschließlich Chlamydia-induzierte Entzündung, durch Virus induzierte Entzündung und Entzündung in Verbindung mit chirurgischen Verfahren, wie z.B. Gefäßtransplantat, einschließlich Koronararterien-Bypass-Operation, Revaskularisationsverfahren, einschließlich Angioplastie, Stentplatzierung, Endarterektomie oder anderer invasiver Verfahren, die Arterien, Venen und Kapillaren involvieren; mit Angiogenese in Verbindung stehende Störungen, z.B. Tumorangiogenese; Neoplasie, einschließlich Metastasierung; ophthalmologischen Zuständen, z.B. Corneatransplantatabstoßung, Augenneovaskularisation, Retinaneovaskularisation, einschließlich Neovaskularisation nach Verletzung oder Infektion, diabetischer Retinopathie, Makuladegeneration, retrolentaler Fibroplasie und Glaukom, einschließlich neovaskulärem Glaukom; Ulkuskrankheiten, z.B. Magenulkus; Hämangiome, einschließlich infantiler Hämangiome, Angiofibrome des Nasopharynx und avaskuläre Knochennekrose; Störungen des weiblichen Fortpflanzungssystems, z.B. Endometriose; benigne und maligne Tumoren, Neoplasie, einschließlich Krebs, z.B. colorektaler Krebs, Gehirnkrebs, Knochenkrebs, von Epithelzellen abgeleitete Neoplasie, z.B. Basalzellenkarzinom, Adenokarzinom, Gastrointestinalkrebs, z.B. Lippenkrebs, Mundkrebs, Ösophaguskarzinom, Dünndarmkrebs, Magenkrebs, Kolonkrebs, Leberkrebs, Blasenkrebs, Pankreaskrebs, Eierstockkrebs, Cervixkrebs, Lungenkrebs, Brustkrebs und Hautkrebs, z.B. Plattenzellkrebs und Basalzellkrebs, Prostatakrebs, Nierenzellkarzinom und andere Krebsarten, die Epithelzellen im Körper befallen; Fibrose, die bei Strahlentherapie auftritt, adenomatöse Polypen, einschließlich familiärer adenomatöser Polypose; Prostanoidinduzierter glatter Muskelkontraktion und mit Eosinophilen in Verbindung stehender Störungen; Knochenverlust, insbesondere bei Frauen nach der Menopause, Osteoporose.

22. Verwendung nach Anspruch 21, wobei das Säugersubjekt ein Mensch ist.

23. Verwendung nach Anspruch 22, wobei das Medikament zur Kombinationstherapie mit einem Wirkstoff oder mehreren Wirkstoffen, ausgewählt aus Opioiden und anderen Analgetika, angepasst ist.

24. Verwendung nach Anspruch 22, wobei das Medikament zur Kombinationstherapie mit einer Opioidverbindung, ausgewählt aus Codein, Meperidin und Morphin, angepasst ist.

## Revendications

1. Produit moulé destiné à être administré dans la cavité orale d'un sujet pour traiter ou prévenir un état, une affection ou une maladie dépendant(e) de la cyclooxygénase-2, le produit moulé comprenant un mélange moulable composé d'une quantité thérapeutiquement efficace du médicament inhibiteur sélectif de la cyclooxygénase-2, le Valdécoxib, et d'un système support d'excipient pharmaceutiquement acceptable comprenant un ou plusieurs hydrate(s) de carbone, dans lequel lesdits hydrates de carbone représentent plus de 50 % en poids de tous les excipients présents dans le mélange moulable, lesdits ingrédients et leurs quantités dans le produit moulé et dans un procédé de préparation du produit moulé étant choisis de sorte que le produit moulé présente une désintégration rapide dans la cavité orale, et le mélange moulable étant préparé par une étape de procédé ne nécessitant pas de granulation par voie humide, ledit objet se désintégrant dans les 30 à 300 secondes suivant la mise en place dans un essai de désintégration standard *in vitro* réalisé en accord avec la pharmacopée américaine N°24 (2000), essai N° 701, le médicament inhibiteur sélectif de la cyclooxygénase-2 étant présent à raison d'une quantité totale de 20 à 60 % en poids du produit moulé.

2. Produit moulé selon la revendication 1, dans lequel le ou les hydrate(s) de carbone présent(s) dans le système support d'excipient est ou sont choisi(s) parmi les celluloses naturelles et modifiées, les amidons naturels et modifiés, les sucres de mono-, di- et oligosaccharides et les alcools de sucres.

3. Produit moulé selon la revendication 1, dans lequel au moins un hydrate de carbone présent dans le système support d'excipient est un sucre ou un alcool de sucre.

4. Produit moulé selon la revendication 3, dans lequel le sucre ou l'alcool de sucre est choisi parmi l'érythritol, le glucose, le lactose, le maltitol, le maltose, le mannitol, le sorbitol, le saccharose et le xylitol.

5. Produit moulé selon la revendication 3, dans lequel le sucre ou l'alcool de sucre présente une dissolution rapide dans la cavité orale d'un sujet et délivre un goût sucré.

6. Produit moulé selon la revendication 1, dans lequel un ou plusieurs hydrate(s) de carbone est ou sont présent(s) à raison d'une quantité totale de 20 à 60 % en poids du produit moulé.

7. Produit moulé selon la revendication 1, qui est un comprimé oral à délitement rapide.

8. Comprimé selon la revendication 7, qui se désintègre dans les 5 à 60 secondes après mise en place dans la cavité orale d'un sujet.

9. Comprimé selon la revendication 7, dont la dureté est comprise entre 9,8 et 98 N (1 à 10 kp).

10. Comprimé selon la revendication 7, présentant une dureté suffisante pour résister à la fracture du comprimé au moment de son extraction de la plaquette thermoformée classique lorsqu'une pression le fait traverser une feuille de protection, où la dureté est supérieure ou égale à 9,8 N (1 kp) pour un comprimé au diamètre approximatif de 8 mm, supérieure ou égale à 14,7 N (1,5 kp) pour un comprimé au diamètre approximatif de 10 mm et supérieure ou égale à 19,6 N (2 kp) pour un comprimé au diamètre approximatif de 12 mm.

11. Comprimé selon la revendication 7, présentant une dureté suffisante pour permettre aux comprimés d'être conditionnés ensemble dans un flacon de verre ou de plastique, sans emballage individuel, les comprimés ne présentant ce faisant aucune fracture sensible ni aucune agglomération et/ou fusion les uns aux autres au cours de l'expédition et de la manipulation normales, la dureté étant supérieure ou égale à 29,4 N (3 kp).

12. Procédé de préparation d'un produit moulé qui est une forme pharmaceutique orale à délitement rapide du valdécoxib, médicament inhibiteur sélectif de la cyclooxygénase-2, le procédé comprenant une étape consistant à mélanger intimement le valdécoxib en quantité thérapeutiquement efficace avec un système support d'excipient comprenant un ou plusieurs hydrate(s) de carbone, pour former un mélange, dans lequel la formation du mélange ne nécessite pas de granulation par voie humide et dans lequel lesdits hydrates de carbone représentent plus de 50 % en poids dudit mélange ; et une étape consistant à mettre en forme une quantité unidose du mélange dans un moule pour former le produit moulé ; ledit produit se désintégrant dans les 30 à 300 secondes suivant la mise en place dans un essai de désintégration standard *in vitro,* réalisé en accord avec la pharmacopée américaine N° 24 (2000), essai N° 701, le médicament inhibiteur sélectif de la cyclooxygénase-2 étant présent à raison d'une quantité totale de 20 à 60 % en poids du produit moulé.

13. Procédé selon la revendication 12, dans lequel le ou les hydrate(s) de carbone présent(s) dans le système support d'excipient est ou sont choisi(s) parmi les celluloses naturelles et modifiées, les amidons naturels et modifiés, les sucres de mono-, di- ou oligosaccharides et les alcools de sucres.

14. Procédé selon la revendication 12, dans lequel au moins un hydrate de carbone présent dans le système support d'excipient est un sucre ou un alcool de sucre.

15. Procédé selon la revendication 14, dans lequel le sucre ou l'alcool de sucre est choisi parmi l'érythritol, le glucose, le lactose, le maltitol, le maltose, le mannitol, le sorbitol, le saccharose et le xylitol.

16. Procédé selon la revendication 14, dans lequel le sucre ou l'alcool de sucre présente une dissolution rapide dans la cavité orale d'un sujet et délivre un goût sucré.

17. Procédé selon la revendication 12, dans lequel un ou plusieurs hydrate(s) de carbone est ou sont présent(s) à raison d'une quantité totale de 20 à 60 % en poids du produit moulé.

18. Procédé selon la revendication 12, dans lequel l'étape de mise en forme comprend une compression directe du mélange pour former un comprimé.

19. Procédé selon la revendication 12, comprenant en outre une étape consistant à débarrasser le produit moulé d'un solvant par cryodessiccation, séchage sous vide ou lyophilisation.

20. Procédé selon la revendication 12, dans lequel le système support d'excipient est préparé sous forme d'une matrice de type shearform à laquelle l'on adjoint le médicament et dans lequel l'étape de mise en forme comprend la compression de la matrice de type shearform.

21. Utilisation d'un produit moulé selon la revendication 1 pour fabriquer un médicament destiné à traiter un état pathologique ou un trouble chez un sujet mammifère pour qui un traitement par un inhibiteur de la cyclooxygénase-2 est indiqué, où le médicament est adapté à l'administration orale et l'état pathologique est choisi dans le groupe constitué par les inflammations, les douleurs, la fièvre, l'arthrite, les affections arthritiques, la polyarthrite rhumatoïde, les spondylarthropaties, l'arthrite goutteuse, l'arthrose, le lupus érythémateux disséminé, l'arthrite juvénile, l'asthme, la bronchite, la dysménorrhée, le travail prématuré, la tendinite, la bursite, la névrite allergique, l'infection par les cytomégalovirus, l'apoptose y compris l'apoptose induite par le VIH, le lumbago, les maladies hépatiques parmi lesquelles l'hépatite, le psoriasis, l'eczéma, l'acné, les brûlures, les dermites, les lésions dues aux rayons ultraviolets parmi lesquelles l'érythème solaire, les inflammations postopératoires y compris celles consécutives à une intervention ophtalmique telle que l'opération de la cataracte ou une opération de réfraction ; les pathologies gastro-intestinales parmi lesquelles les infections inflammatoires du tube digestif, la maladie de Crohn, la gastrite, la colopathie fonctionnelle, la rectocolite hémorragique ; des inflammations telles que dans certaines maladies comme les migraines, la périartérite noueuse, la thyroïdite, l'anémie aplasique, la maladie d'Hodgkin, la sclérodermie, les rhumatismes articulaires aigus, le diabète de type I, les maladies de la jonction neuromusculaire telles que la myasthénie grave, les maladies de la substance blanche parmi lesquelles la sclérose en plaques, la sarcoïdose, le syndrome néphrétique, le syndrome de Behçet, la polymyosite, la gingivite, la néphrite, l'hypersensibilité, le gonflement faisant suite à une blessure tel qu'un oedème cérébral, une ischémie myocardique ; les maladies ophtalmiques, telles que la rétinite, la sclérite, l'épisclérite, la conjonctivite, les rétinopathies, l'uvéite, la photophobie oculaire, les lésions aiguës touchant les tissus oculaires ; les inflammations pulmonaires telles que celles associées aux infections virales et à la mucoviscidose ; les troubles du système nerveux central, tels que les démences corticales parmi lesquelles la maladie d'Alzheimer, la neurodégénérescence, et les lésions du système nerveux central consécutives à une attaque cérébrale, une ischémie ou un traumatisme ; les démences, parmi lesquelles la démence vasculaire, la démence athéroscléreuse, la démence présénile, la démence alcoolique et la démence sénile ; la rhinite allergique, le syndrome de détresse respiratoire, le syndrome du choc endotoxinique ; les douleurs, parmi lesquelles la douleur postopératoire, la douleur dentaire, la douleur musculaire et la douleur d'origine cancéreuse ; les douleurs, la fièvre et l'inflammation dans des états comprenant les rhumatismes articulaires aigus, la grippe et autres infections virales parmi lesquelles le rhume banal, les lombalgies basses et les cervicalgies, la dysménorrhée, les maux de tête, les maux de dents, les entorses et les foulures, la myosite, les névralgies, la synovite, l'arthrite, y compris la polyarthrite rhumatoïde, les arthroses (ostéo-arthrose), la goutte et la spondylite ankylosante, la bursite, les brûlures et les traumatismes post-chirurgicaux ou post-dentaires ; les troubles cardiovasculaires associés à une inflammation ; les maladies vasculaires, l'insuffisance coronarienne, l'anévrisme, le rejet vasculaire, l'artériosclérose, l'athérosclérose, dont l'athérosclérose de transplantation cardiaque, l'infarctus du myocarde, l'embolie, l'attaque cérébrovasculaire, la thrombose, y compris la thrombose veineuse, l'angor, y compris l'angor instable, les inflammations de la plaque coronaire, les inflammations d'origine bactérienne telles que les inflammations dues aux Chlamydia, les inflammations d'origine virale et les inflammations associées aux opérations chirurgicales telles que les greffes vasculaires parmi lesquelles la chirurgie de pontage aorto-coronaire, les opérations de revascularisation parmi lesquelles l'angioplastie, la pose d'extenseurs (stenting), l'endartériectomie ou autres procédures invasives mettant en cause les artères, les veines et les capillaires ; les troubles associés à l'angiogenèse, tels que l'angiogenèse tumorale ; la néoplasie, y compris la dissémination métastasique ; les problèmes ophtalmologiques tels que le rejet de greffe de la cornée, la néovascularisation oculaire, la néovascularisation rétinale y compris la néovascularisation découlant d'une blessure ou d'une infection, la rétinopathie diabétique, la dégénérescence maculaire, la fibroplasie rétrolentale et le glaucome, y compris le glaucome néovasculaire ; les maladies ulcératives telles que l'ulcère gastrique ; les hémangiomes, y compris les hémangiomes infantiles, l'angiofibrome du rhinopharynx et la nécrose avasculaire osseuse ; les affections de l'appareil génital féminin telles que l'endométriose ; les tumeurs/néoplasies bénignes et malignes y compris les cancers, tels que le cancer du côlon et du rectum, le cancer du cerveau, le cancer des os, la néoplasie dérivée de cellules épithéliales telle que le carcinome basocellulaire, l'adénocarcinome, le cancer gastro-intestinal tel que le cancer des lèvres, le cancer de la bouche, le cancer de l'oesophage, le cancer de l'intestin grêle, le cancer de l'estomac, le cancer du côlon, le cancer du foie, le cancer de la vessie, le cancer du pancréas, le cancer de l'ovaire, le cancer du col de l'utérus, le cancer du poumon, le cancer du sein et le cancer de la peau, tel que l'épithélioma spinocellulaire et l'épithélioma basocellulaire, le cancer de la prostate, le carcinome des cellules rénales et autres cancers connus qui affectent les cellules épithéliales à travers l'ensemble de l'organisme ; la fibrose découlant d'une radiothérapie ; les polypes adénomateux, y compris la polypose adénomateuse familiale ; la contraction des muscles lisses à induction prostanoïde et les anomalies d'origine éosinophile ; la déminéralisation osseuse, en particulier chez la femme postménopausée, l'ostéoporose.

22. Utilisation selon la revendication 21, ledit sujet mammifère étant un patient humain.

23. Utilisation selon la revendication 22, le médicament étant adapté à une polythérapie associant un ou plusieurs médicament(s) choisi(s) parmi des opioïdes et autres analgésiques.

24. Utilisation selon la revendication 22, le médicament étant adapté à une polythérapie associant un composé opioïde choisi parmi la codéine, la mépéridine et la morphine.
